# EUROPEAN PATENT SPECIFICATION

(11) **EP 1 339 425 B1**
(45) Date of publication and mention of the grant of the patent: **19.08.2009**
(21) Application number: 01999383.1
(22) Date of filing: 06.12.2001
(51) Int. Cl.: A61K 38/18, A61K 39/00, A61K 39/385, A61K 47/48, C07K 14/495

(54) **VACCINE COMPOSITION CONTAINING TRANSFORMING GROWTH FACTOR ALPHA**
TRANSFORMIERENDER WACHSTUMSFAKTOR ALPHA ENTHALTENDE IMPFSTOFFZUSAMMENSETZUNG
COMPOSITION DE VACCIN CONTENANT LE FACTEUR DE CROISSANCE TRANSFORMANT ALPHA

(30) Priority: 06.12.2000 CU 2862000
(43) Date of publication of application: 03.09.2003
(73) Proprietor: Centro de Inmunologia Molecular, 16040 Provincia Ciudad Habana (CU)
(72) Inventor: MULET SIERRA, Aillette, 10800 Provincia Ciudad Habana, Cuba (CU); PEREZ RODRIGUEZ, Rolando, 10500 Provincia Ciudad Habana, Cuba (CU); GONZALEZ MARINELLO, Gisela Maria, 11600 Provincia Ciudad Habana, Cuba (CU); ALVAREZ ACOSTA, Anabel, 12100 Provincia Ciudad Habana, Cuba (CU); MENENDEZ MEDINA, Tamara, 11300 Provincia Ciudad Habana, Cuba (CU); GUILLÉN NIETO, Gerardo Enrique, 10400 Ciudad de la Habana, Cuba (CU); SANCHEZ RAMIREZ, Belinda, Centro Habana, Provincia Centro Habana (CU)
(74) Representative: Hatzmann, Martin
(86) International application number: PCT/CU2001/000011
(87) International publication number: WO 2002/045738

(56) References cited:
- WO-A-91/01146
- GB-A- 2 333 706
- US-A- 5 690 928
- US-A- 5 894 018
- D'ERRICO A ET AL.: "Role and new perspectives of transforming growth factor-alpha (TGF-a1) adenocarcinoma of the gastro-oesophageal junction." BR J CANCER, vol. 82 (4), February 2000 (2000-02), pages 865-870, XP002210096

## Description

### Technical Sector

The present invention relates the field of immunology and human medicine, in particular with a vaccine preparation able to provoke an immune-castration reaction against the autologous TGFα. This vaccine can be used for the treatment of certain cancer and other TGFα related diseases.

### Prior Art

The transforming Growth Factor (TGFα) is a 50 amino acids polypeptide, originally isolated of the conditioned medium of retrovirus-transformed cells. At the beginning it was defined like a molecule able to compete with the Epidermal Growth Factor (EGF) for EGF-R binding. However, antibodies anti-EGF were not able to recognize TGFα (Todaro et al. (1976), Nature 264, 26-31). Then, both growth factors are two immunologically different entities.

TGFα belong to the EGF family. Structural and functional related proteins constitute this family. The other members of this family are EGF, amphiregulin (AR), criptol (CR1), heparin-binding EGF, betacellulin, epiregulin. On the other hand the poxvirus family includes EGF related proteins. Inside them the most characterized is the vaccinia virus growth factor (VGF).

All these molecules bind and activate EGF-R, that's why they are known as ligands of this receptor. This system plays a role in the growth of normal and neoplastic cells.

EGF-R is a glycoprotein of 170 kD whose gene has been cloned and sequenced. The intracellular domain of this receptor is associated with the tyrosine kinase proteins activity. These proteins show structural homology with the v-erb-B oncogen product, what is an evident of a relationship with the neoplastic transformation process (Heldin C.H. (1984), Cell 37, 9-20.)

TGFα is synthesized as transmembrane precursor form (pro-TGFα) of 160 amino acids. Mature TGFα, a soluble form of 50 amino acids, is released by proteolytic cleavage. Human TGFα (hTGFα) shows 43% amino acid sequence identity with human EGF (hEGF) and 93% with mouse or rat TGFα. Besides, their biological effects are not species-specific.

The work of many laboratories has documented the ability of TGFα to regulate the proliferation, migration and differentiation of cells in culture (Carpenter and Wahl. (1990), Springer-Verlag, Berlin, pp.69-171.)

TGFα is the most widespread ligand of EGF-R. It is expressed in normal tissues during the embriogenesis and in normal and tumor tissues in adults. However, not major pathological defects are observed in TGFα knockout mice and these mice are viable and fertile (Bruce Mann and cols. (1993), Cell, 73, 249-261.).

In the tumorigenesis process, the deregulation of paracrine and autocrine processes of activation of EGF-R is due to the up-regulation of growth factor expression or the high synthesis or mutation of their receptor.

In epithelial tumors high levels of EGF-R have been detected. In many cases, the over-expression of this receptor constitutes an indicator of bad prognosis.

The induction of TGFα is a frequent event in neoplastic transformation. In fact, there are numerous studies that show the over-expression of this molecule in epithelial tumors of different localizations that include, breast, lung, brain, liver, prostate, bladder, gastrointestinal tract, colon, reproductive (ovary) and endocrine tissues, among others.

Although the mechanism by which TGFα induce tumorigenicity remain unknown, there are some reports that correlate the over-expression of this growth factor with tumor degree, survival of patient and other tumoral markers. Besides, some researchers have demonstrated their relationship with other oncogenes like c-myc in hepatocarcinomas. TGFα also constitutes a target of the von Hippel-Lindau tumor suppressor gene (VHL) (summarized in Lee et al. (1996), Growth Factors and Cytokines in Health and Disease, Volume 1B, 277-318.).

Although TGFα and EGF bind the same receptor with comparable affinities, TGFα is generally more potent than EGF, and in some contexts, its effects have been described as stronger and/or more prolonged (Barrandon and Green (1987), Cell 50, 1131-1137). It has been reported that in the case of an internalized TGFα/EGF-R complex, TGFα and EGF-R were preferentially recycled back to the cell surface while when an EGF/EGF-R complex were internalized in the same cell type, both components were efficiently degraded (Ebner and Derynck (1991), Cell Regul.2, 599-612). These results suggested that the differences in biological activity of each growth factor might be due to differences in the intracellular trafficking mechanisms.

On the other hand TGFα is a more potent angiogenic factor than EGF (Schreiber et al. (1986), Science 232, 1250-1253.)

Concerning to the expression in tumors, there are evidences of the presence of EGF precursor in membranes of some epithelial tumors but TGFα is most express in epithelial tumors, and its action, contrary to EGF, is by an autocrine loop with the EGF-R. On the other hand, results of our center indicate that in some epithelial tumor biopsies there are expression of TGFα and non EGF (breast ductal carcinoma, larynx carcinoma), while other tumors present more EGF than TGFα (non small cell lung cancer (NSCLC)). These results suggest that growth factors can have different impacts in the tumor biology of different neoplastic cells.

All the evidences accumulated in these years about the relationship among the system EGF-R / EGF-R ligands and cancer, convert this system in a very attractive target for the cancer immunotherapy.

Previous results of our group have demonstrated the possibility to develope an active cancer immunotherapy with a vaccine based on EGF. In fact, preclinical and clinical evidences have been obtained about the immunogenicity and low toxicity caused by the vaccination with hEGF coupled to a carrier protein (González et al. (1996), Vaccine Research 5(4), 233-243.)

Preclinical studies have shown that immunization of mice with hEGF in adjuvant increases the survival of mice transplanted with Ehrlich ascites tumor (EAT) (González et al. (1996), Vaccine Research 5(4), 233-243.).

A fusion protein between hEGF and P64k was produced. This protein contains the hEGF sequence inserted between amino acids 45/46 of P64k. This fusion protein was used to inmunize mice, causing a specific humoral immune response against hEGF. The immune response generated provoke an increase of life span of EAT bearing mice (González and cols (1997), Vaccine Research 6(2), 91-100).

In two pilot clinical trials with patient of NSCLC, was observed an trend to increase the survival in vaccinated patients compared with an historical control. In patients with high antibody response against the hEGF a marked increase of survival was observed (González et al. (1998), Annals of oncology 9, 1-5.).

In general the vaccination with EGF doesn't generate a specific antibody response against TGFα. However have been obtained evidences that the vaccination with an immunogenic preparation containing TGFα in a murine model generates low levels of anti-EGF antibodies only in some mice. This antibody response in some cases is able to block the EGF binding to its receptor *in vitro*. However the levels of anti-EGF antibodies obtained are not enough to generate an effective EGF immune-castration response with impact in the anti-tumoral action.

Due to the action of each of these growth factors is different in each tumor and/or between the primary tumor and its metastasis, a vaccine that combines the two main ligands of the EGF-R, TGFα and EGF, have a better anti-tumoral effect in epithelial tumors, in general sense.

Until the moment of the present invention, has not been developed any therapy that proposes the use of a vaccine preparation containing hTGFα or any derived, or a combination with other ligand of the EGF-R, EGF, in the active cancer immunotherapy.

### Disclosure of the Invention

The present invention provides a vaccine composition to elicit an immune response against autologous TGFα that contains as active principle, a fusion protein between a carrier protein and human TGFα or any peptide derivative of the same and additionally comprising an adjuvant, wherein said fusion protein is able to elicit the immune response against TGFα. The vaccine is able to inhibit the growth of epithelial tumors without adverse collateral effects.

This action is through a growth factor immune-castration mechanism. It also claims a vaccine composition that further contains EGF and a carrier protein.

The vaccine composition can be used in the treatment of epithelial tumors dependent of TGFα or TGFα/EGF, or in any other disease associated with TGFα such as psoriasis (Kapp et al (1993) J Dermatol Sci, Jun;5(3):133-42).

In the specification of TGFα, any fragment derived of TGFα that has the same immunology properties and/or similar effects to the original molecule is included.

Those derived include, but they are not excluded other, original substitutions of amino acids, change of specific amino acids that increase the stability and/or the activity, chemical modifications, among others.

More specifically the invention consists on a vaccine composition able to cause an immune-castration reaction of self-TGFα that can be used for the treatment of certain cancers and other diseases related with TGFα.

On the other hand this invention includes the use of a vaccine preparation constituted by a combination of TGFα and EGF. This vaccine can be used for the treatment of neoplasias that depend on these two growth factors in the course of its pathogenesis. Vaccine composition may be used in the treatment of malignant disease in tumours of epithelial origin which express TGFα or EGF such as an epidermoide carcinoma of the lung, breast, prostate, gastric or ovary.

### 1 - Immunogenic preparations:

In the present invention a vaccine preparation used includes a fusion protein between the hTGFα and a carrier protein. The hTGFα used in any one of these immunogenic preparations can be recombinant, synthetic or obtained from natural source. Different proteins can be used as carriers. An example of a carrier protein that can be used is P64k protein from the outer membrane protein complex of *Neisseria meningitidis* and may comprise a tail of six histidines in the N-terminal end. The optimum quantity of hTGFα in the vaccine formulation oscillates between 5 µg and 1000 µg per dose. The adjuvant maybe Incomplete Freud Adjuvant or Al(OH)₃.

On the other hand a vaccine preparation that contains a combination of hTGFα with hEGF (Office of National Registration of Medications, HEBERMIN Not 1266) is used.

In the specification of TGFα or EGF, any fragment derived from TGFα or EGF that has the same immunology properties and/or similar effects to the original molecule is included. Those derived include, but they are not excluded other, original substitutions of amino acids, change of specific amino acids that increase the stability and/or the activity, chemical modifications, among others.

### A) Obtaining of a fusion protein TGFα-carrier protein by genetic engineering methods:

The gene coding for hTGFa (500 pb) was amplified by polymerase chain reaction (PCR) using specific primers. The resulting DNA fragment is digested and cloned in a specific site to an expression vector containing the gene coding for the carrier protein. The resulting protein includes a single or multiple copies of both molecules. You can use an expression vector of mammalian cells, bacteria or yeast. The vector can also include six histidines in the N-terminal end of the carrier protein. The resulting plasmid is verify by restriction analysis on agarosa gels, DNA sequencing using Sequenase 2.0 (Amersham-USB), and finally, analysis of expression of fusion protein in any E. coli expression strain by Western Blott technique, using an antibody specific monoclonal against hTGFα (R&D System). To obtain the protein the bacterial walls is disrupted using a strong rupture method and then the protein are becomes purified for a combination of differential precipitation methods with ammonium sulfate and chromatography methods. Finally, the protein is filtered under sterile conditions and conserved to -20°C or lyophilized and conserved at 4°C until its later use.

### B) Obtaining of a chemical conjugated containing hTGFα:

Different preparations that contain hTGFα conjugated with different carrier proteins (as P64k) are obtained. Any chemical conjugation method can be used. As preferential chemical method is used the method using EMCS agent described in the North American patent, U.S.Pat, Not. 4,302,386; Lee et al., 1981.

Alternatively, you can use the conjugation method with glutaraldehyde. Briefly, these two or three molecules to a concentration of 1 mg/mL in the final solution are mixing with glutaraldehyde to 0.05% (in the total solution). The mixture is incubated for 1 hour at room temperature and then dialyzed against a solution of PBS 1X/10 mM MgCl₂. Finally, a dialysis against PBS 1X is carried out overnight at 4°C. The inmunogenic preparation is filter under sterile conditions and stored at 4°C until its use.

### C) Obtaining of a vaccine that combines hTGFα and hEGF.

The obtaining of a vaccine that combines the two main ligands of the EGF-R can be performed in different ways:
1 Mixing the two vaccines that contain hTGFα or hEGF for separate linked to a carrier protein in a relationship 1:1 just in the moment of the injection. For this purpose can be used the fusion proteins or those chemistry conjugated of each growth factor and carrier protein. The optimum quantity of hTGFα and hEGF in the vaccine formulation oscillates between 5 µg and 1000 µg per dose.
2 Obtaining of a similar genetic construction to the one described in the section A containing both growth factors, hTGFα and hEGF or a combination of anyone of their derived.
3 Chemical obtaining of a chemist conjugated containing hTGFα and hEGF or a combination of anyone of their derived and a carrier protein using the methodology described in the section B.

### D) Obtaining of the immunogenic preparation:

To obtain the desired immunogenic effect of the vaccine compositions is convenient to use an appropriate adjuvant and to select an administration route in which the vaccine preparation exhibits a high immunogenicity.

The vaccine compositions referred in this invention are prepared in two specific ways:
1) Using Al(OH)₃ as adjuvant to obtain a watery solutions from the vaccine preparation adsorbed to this compound. For this purpose is used a range from 5 µg to 1000 µg of TGFα equivalent in the different preparations bound to a range from 2 to 5 mg of Al(OH)3. This formulation is left in agitation for 1 hour. The final solution is conserved at 4°C until its later use.
2) Using incomplete adjuvant of Freund that allow the formation of aqueous/oil or oil/aqueous emulsions. The quantities of fusion protein or chemist conjugated and adjuvant in the final formulation are in a range from 40/60 to 60/40 (volumelvolume). The volumes depend on the final emulsion desired. The adjuvant is added before the immunization and the formulation is agitated for a period from 10 to 30 minutes, at room temperature.

The final volumes of each immunogenic preparation cover the appropriate range for the corresponding route of administration.

In the case of the combined vaccines prepared just in the moment of the injection as was described in the section C, the two vaccine mixed with the appropriated adjuvant like it has been described previously can be mixed by agitation and inject or inject separate.

The administration of the vaccine compositions can be done for diverse routes: intramuscular, subcutaneous, intranasal and intradermal.

### Examples

### Example 1: Obtaining the DNA segment coding for mature TGFα by polymerase chain reaction (PCR).

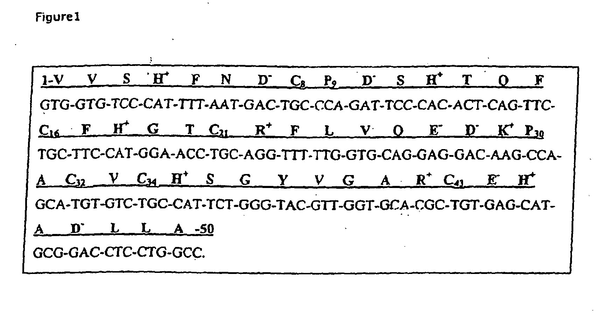

The gene coding for hTGFα was amplified by PCR using as template the PSK/TGFα vector (CIGB, Cuba). That plasmid contains the hTGFα complementary DNA (cDNA) cloned in the EcoR V site of commercial vector pbluescript KS - (Stragene). The sequence coding for the mature TGFα (50 amino acids long (Fig.1)) was amplified using the specific primers describe above:
N-Terrminal: 5' - GCTCTAGAAGTGGTGTCCCATTTTAATGAC-3'
   (Underlined, Xbal restriction site)
C-terminal: 5'-CGGAATTCGCCAGGAGGTCCGCATGCTCAC-3'
   (Underlined, EcoRI restriction site)

Briefly, 200 ng of the PSKTGFα was used in 75 µL of a mixture that contains: 500 ng of each one of the specific primers, a mixture of deoxynucleotide triphosphates to a concentration of 200 mM each one, 25 mM MgCl₂ and 100 Units of Taq-Polimerase enzyme (Promega) in buffer solution given by Promega. A protocol of 30 cycles of denaturalization (1 minute at 94°C), annealing (1 minute at 60°C), and extension (30 seconds at 72°C) was followed. Before the first cycle, DNA was denatured for 4 minutes and after the last cycle; a final extension of 2 minutes was performed.

PCR product is electrophoretically separated on low gelling temperature (LGT) agarose gels and the amplified gene segment becomes purified according to conventional procedures of extraction with phenol and enzymatic digested using Xba I and EcoR I enzymes (NEB, USES). Following this protocol is prepared the gene segment coding for the mature TGFα.

### Example 2: Obtaining of the expression vector for the fusion protein TGFα-P64K.

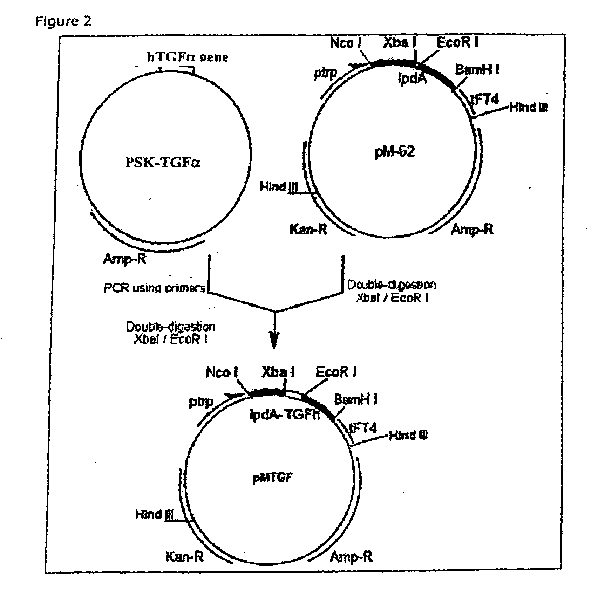

The expression vector pM 92 was used (CIGB, Cuba). This plasmid contains the IpdA gene coding for P64k protein from *Neisseria meningitidis* (strain B385) under the control of E. coli tryptophan operon promoter (ptrp) and phage T4 transcriptional terminator (tT4). pM 92 code for ampicillin and kanamicin antibiotic resistance. A Dam - E. coli-strain (GC-366) is transformed with pM92 and the plasmid is purified using a commercial kit (Quiagen) according to the protocol of the manufacturer. PM92 vector were digested and purified from LGT agarose gels. Subsequently PM92 vector is ligated with the cDNA from mature hTGFα previously prepared, using T4 ligase enzyme (Gibco BRL). The resulting plasmid pMTGFα codes for the fusion protein that contains hTGFα inserted among the amino acid 45/46 of P64k. This recombinant plasmid was verified by restriction analysis in agarose gels, DNA sequencing using Sequenase 2.0 (Amersham-USB), and finally, analysis of the fusion protein expression in E.Coli MM299 strain by Western Blotting technique using a monoclonal antibody specific for hTGFα (R&D System). The figure 2 shows a schematic representation of the expression vector pMTGFα obtaining process. This vector codes for the fusion protein TGFα-P64K.

### Example 3: Obtaining of the expression vector of fused protein TGFα-P64K with six histidines in the N-terminal end (pMHisTGFα).

The expression vector pMHisTGFα was obtained following the same protocol described in the previous example using as starting vector pM224, that includes a segment coding for six histidines in the N-terminal end of P64k. The six histidines tag present advantages in the purification of the protein because increment the binding to chelating Sepharose charged with Cu2+ or other metals.

### Example 4: Fusion protein (TGFα-P64K) purification.

E. coli bacteria (strain MM299) expressing the fusion protein TGFα-P64K were grown in LBA medium (10 g/L Triptone, 5g/L Yeast Extract, 10 g/L NaCl and 50 mg/L ampicillin) for 10 hours at 37°C. After cells collection, all steps were performed at 0-4°C. Bacterial disruption was achieved in a French press at 1500 kg/cm², and the insoluble fraction was removed by high-speed centrifugation for 30 minutes at 11,000 xg. As first purification step a 40% of ammonium sulfate precipitation was done to remove part of the E. coli proteins. The resulting pellet was removed by a further centrifugation at 4°C for 30 minutes to 11,000 x g. The supernatant was fractioned by hydrophobic interaction chromatography (TSK-butil, Pharmacia, Sweeden), with a decreasing gradient of ammonium sulfate from 40% to 0% in buffer Tris-Cl, pH = 7.2 containing 0.15M NaCl. Subsequently the resulting sample was separated by gel filtration on a G200 column (Pharmacia) equilibrated with PBS 1X, achieving a final purity of more than 95%. Proteins concentration is determined using a colorimetric method described by Lowry et al (1951) J.Biol.Chem. 191, 495-498). The characterization of fused protein was done by Western Blotting technique, using specific antibodies against P64k and TGFα.

### Example 5: Fusion protein (HisTGFα-P64k) purification.

E. coli bacteria (strain MM299) expressing the fusion protein TGFα-P64K were grown in LBA medium (Triptone 10 g/L, Yeast Extract 5g/L, NaCl 10 g/L and 50 mg/L ampicillin) for 10 hours at 37°C. After cells collection, all steps were performed at 0-4°C. Bacterial disruption was achieved in a French press at 1500 kg/cm², and the insoluble fraction was removed by high-speed centrifugation for 30 minutes at 11,000 xg. As first purification step a 40% of ammonium sulfate precipitation was done to remove part of the E. coli proteins. The resulting pellet was removed by a further centrifugation at 4°C for 30 minutes to 11,000 x g. The supernatant was fractioned by chelating affinity chromatography (Chelating Sepharose Fast Flow, Pharmacia, Sweeden), due to the presence of six histidines in the protein, with a increasing Imidazol gradient from 25 mM to 500 mM in buffer Tris-Cl, pH = 5.5 containing 0.5 M NaCl. Subsequently the resulting sample was passed through on gel filtration G25 column (Pharmacia) equilibrated with PBS 1X to remove the salts, achieving a purity level of 95%. Proteins concentration is determined using a colorimetric method described by Lowry et al (1951) J.Biol.Chem. 191, 495-498). The characterization of the fused protein was done by Western Blotting technique, using specific antibodies against P64k and TGFα.

### Example 6: Recognition of the recombinant protein TGFα-P64K for a monoclonal antibody (Mab) specific for hTGFα.

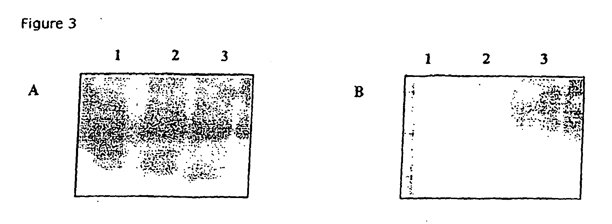

To determine if TGFα could be recognized by an anti-hTGFα Mab (Calbiochem) in the fused protein context, was done a Western Blotting technique. Electrophoretically 25 µg of EGF-P64K, TGFa-P64K or P64K were separated in two polyacrilamide gels and then transferred to a 0.45 µm nitrocellulose membrane according to conventional procedures. After the transfer, membranes were incubated with a blocking solution of TBS 1X with 5% of skim milk overnight at 4 °C. After a brief wash with TBS 1X-Tween 20 (0.05%), membranes were incubated, one replies with an antibody anti-P64K (1 / 500) (Fig. 3A) and the other one with a anti-TGFα Mab (1/100) (Fig. 3B) for 2 hours at room temperature. Subsequently were performed 3 washes with the same solution and membranes were incubated with alkaline phosphatase-labeled goat anti-mouse immunoglobulins (1/1000) for 1 hour in same conditions. Finally was added 0.004 g of Fast Net enzyme substrate (Sigma) in buffer containing 0.1 M Tris-Cl pH=8.2, 0.004 g of Naphtol ACE-MX Phosphate (Sigma) and 400 µL of NN'Dimetil Formamide in 20 mL. The reaction stopped with similar washes. A specific recognition of TGFα-P64k by the anti-hTGFα Mab was observed (Fig. 3). This result demonstrates that TGFα in the fusion protein maintains a structure able of being recognized by a specific antibody.

### Example 7: Obtaining of a chemical conjugated hTGFα-P64k.

A milliliter of TGFα in PBS/10 mM MgCl₂ at 2 mg/mL is mixed with a milliliter of P64k at 2 mg/mL in the same solvent. Then 0.2 mL of 0.5% glutaraldehyde solution was added for a final percent of 0.05%. The mixture was incubated 1 hour at room temperature, and dialyzed against a PBS 1X/10 mM MgCl₂ solution. Finally, dialysis against PBS 1X was carried out overnight at 4°C. The immunogenic preparation is filtered under sterile conditions and stored at 4°C until its use.

### Example 8: Obtaining of a fusion protein between hTGFα, hEGF and P64k.

The gene coding for hEGF (150 pb) it is amplified by PCR using the plasmid pBEF 10 as template. That plasmid contains the complete hEGF cloned in the EcoR V site of commercial vector pbluescript SK II (Stragene). The obtained DNA is linked to the pMHisTGFα plasmid in a Bam HI site located in the C-terminal end of the P64k using the methodology described in the example 2. This way the pMTGFα-EGF vector is obtained that codes for the fusion protein TE-P64k.

### Example 9: Obtaining of a chemical conjugated hTGFα-hEGF-P64k.

A milliliter of TGFα in PBS/10 mM MgCl₂ at 3 mg/mL is mixed with a milliliter of hEGF at 3 mg/mL and P64k at 3 mg/mL in the same solvent. Then 0.6 mL of 0.5% glutaraldehyde solution was added for a final percent of 0.05%. The mixture was incubated 1 hour at room temperature, and dialyzed against a PBS 1X/10 mM MgCl₂ solution. Finally, dialysis against PBS 1X was carried out overnight at 4°C. The immunogenic preparation is filtered under sterile conditions and stored at 4°C until its use.

### Example 10: Preparation of formulations that contain hTGFα.

The different immunogenic preparation described in the examples 2, 3, 7, 8 and 9 are mixed with Al(OH)₃ or Montanide ISA 51 as was described in detailed description of the invention. Quantities used that are equal to 50µg of hTGFα in all the preparations and 50µg hTGFα and hEGF, in the case of the combined vaccines described in the examples 8 and 9. Two milligrams of Al(OH)₃ was used by each preparation of fused protein or chemist conjugated containing respectively 50µg hTGFα or hEGF equivalent.

### Example 11: Preparation of a combined vaccine containing TGFα-P64K protein and EGF-P64K protein.

Fifty micrograms of each growth factor in 0.6 mg of recombinant are mixed in a total volume of 0.5 mL with same volume of Montanide ISA 51 and agitated for 10 minutes at room temperature before the injection.

In the case of using Al(OH)₃ as adjuvant, two preparations containing 0.6 mg of each protein in 2 mg of Al(OH)₃ are mixing before the injection.

### Example 12: Preparation of a combined vaccine containing a chemical conjugated TGFα/P64K and EGF/P64K.

An immunogenic preparation containing 50 µg of hTGFα linked to P64k as described in the example 7 in 0.25 mL is mixed with 0.25 mL of an equals immunogenic preparation that contains hEGF and mixed with 0.5 mL of Montanide ISA 51 according was described in the example 10, using a syringe, for a period of 10 minutes at room temperature.

In the case of using Al(OH)₃ as adjuvant, 0.5 mL of each one of those chemical conjugated described before that contain 50µg of hTGFα or hEGF respectively adsorbed in 2 mg of Al(OH)3 are mixed.

### Example 13: Immunogenicity of TGFα-P64K / Incomplete Freund adjuvant (Montanide ISA 51) in a murine model.

To demonstrate the immunogenicity of the vaccine, Balb/c mice, females among 6-8 weeks, were injected subcutaneous with 58 mg (5µg equivalent of TGFα), 116mg (10µg) or 0,6 mg (50µg) of TGFα-P64k with Montanide ISA 51 in a 1:1 proportion. The immunogen was prepared as described in the detailed description of the invention and agitated for 10 minutes before the immunization. Each animal received 4 doses. The blood was extracted before the immunization, one week later and biweekly since that moment. The serum was separated from the extracted blood of animals and specific antibodies titer was determined against the hTGFα by an indirect ELISA technique.

Briefly, microtitter ELISA's plates (COSTAR) were coated with 50µL/well of an hTGFα solution at 2.5 µg/mL in buffer carbonate-bicarbonate pH= 7.2 and incubated overnight at 4°C. After three washes with PBS 1X-Tween 20 (0.05%), the plates were blocked with a solution of PBS 1X-Tween 20 (0.05%) -SFT (5%) for 1 hour at 37°C. Immediately the serums of the immunized mice were added and incubated for 2 hours at 37°C. After washing, the plates were incubated with alkaline phosphatase-labeled goat anti-mouse immunoglobulins (Sigma) diluted 1/1000 in PBS 1X-Tween 20 (0.05%) -SFT (5%) (50µL /pozo) for 1 hour at same temperature. Finally, after washing, the substrate of the enzyme (p-nitrophenylphosphate(Sigma)) was added to final concentration of 1 mg/mL in buffer Dietanolamine pH=9.8 (50µL /well). The absorbance at 405 nm of enzyme-substrate complex formed was measured in an ELISA plate reader.

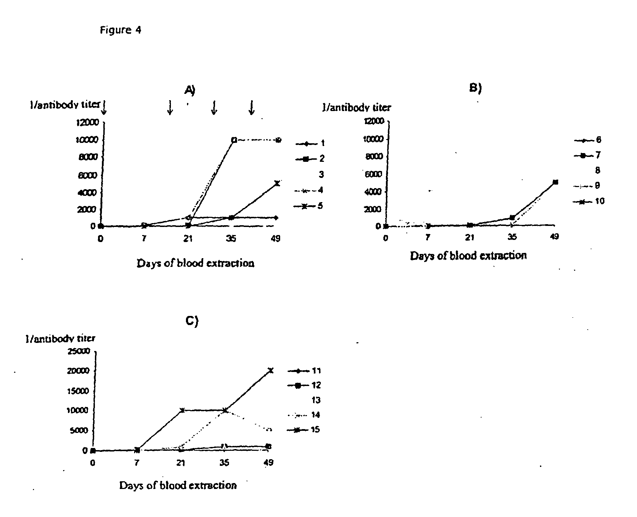

The figure 4 show the kinetics of the polyvalent anti- hTGFα antibody response obtained in mice immunized with TGFα-P64K.

Due to the high homology between hTGFα and rat or mouse counterpart (93%) you can consider this immune response against hTGFα as a response against self-TGFα (murine).

### Example 14: Inmunogenicity of TGFα-P64K / Al(OH)3 in a murine model.

An immunization protocol was done according was described in the previous example, using 2 mg Al(OH)₃ as adjuvant. The immunogenic preparation got ready according was described in the detailed description of the invention. Antibody titers up to 1/10000 were obtained for TGFα. The technique used to determine the antibody titers was the indirect ELISA described in the example 13.

### Example 15: IgG Subclass distribution in mice immunized with TGFα-P64k protein / incomplete Adjuvant of Freund (Montanide ISA 51) in a murine model.

The IgG subclass distribution was determined by an indirect ELISA'S technique described in the example 13, using specific antiserum against the different IgG subclasses conjugated with biotin (Jackson) to a dilution of 1/1000 and later on the complex streptavidine-phosphatase (1/1000).

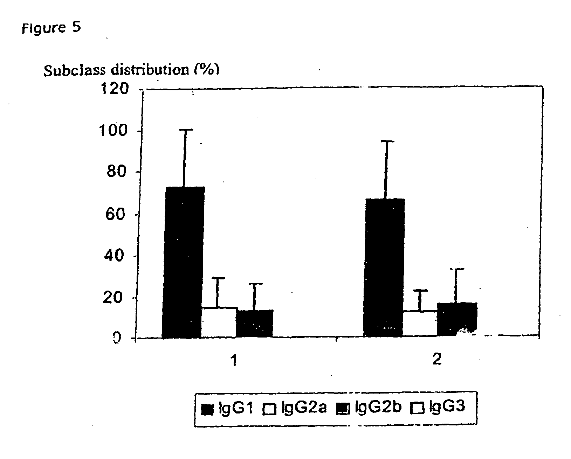

The proportion of each IgG subclass was determined regard to total IgG in the serum of immunized animals. The animals were immunized with 50 µg of TGFα in the fused protein using subcutaneous (Group 1) or intramuscular (Group 2) route following the immunization protocol described in the example 13. In the figure 5 is observed the subclass distribution obtained. A bigger proportion of IgG1 was obtained in both groups of mice used in the study.

### Example 16: Determination of the capacity of animal serums immunized with TGFα-P64k protein / incomplete Adjuvant of Freund (Montanide ISA 51) of inhibit the binding of I₁₂₅-TGFα by radio receptor assay technique (RRA).

To determine if the antibodies generated in the immunization protocols described previously were able to inhibit the TGFα binding to its receptor, was done an in vitro technique called RRA. In synthesis, the serums of immunized mice obtained as it is described in the example 13 were incubated with a mixture that includes 100 mL of human placenta membrane and 20 mL of I₁₂₅-TGFα (100000 cpm) and 330 mL of buffer: 10 mM Tris-Cl, 10 mM MgCl₂ and BSA to 1%, pH=7.4. TGFα was coupled to ₁₂₅I radioisotope using the method of chloramine T (Hunter and Greenwood (1962, Nature, 358:495-498). The mixture was incubated by 1 hour at room temperature and the reaction was stopped with 1 mL of the buffer mentioned before. Finally the tubes were centrifuged at 1000 rpm for 30 minutes. Pellets were washed and allowed to dry off. The radioactivity was measured in a gamma emission counter (Wallac, Finland). The decrease in the measured values of radioactivity indicates the inhibition of binding between TGFα and its receptor, due to the action of tested serums. A range of 50%-80% inhibition percent was obtained among all tested serums.

### Example 17: Determination of the anti-human EGF (hEGF) humoral response generate by the immunization with TGFα-P64k protein.

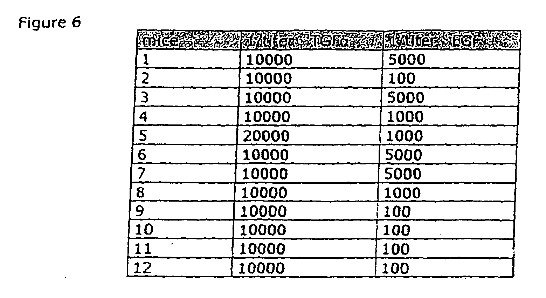

The presence of anti-EGF antibodies was determined in serums of mice that showed high anti-TGFα antibodies titers using the described indirect ELISA'S technique. Dilutions of 1/100, 1/1000 and 1/10000 of serums were added to plates coated with hEGF (CIGB). The figure 6 shows the anti-EGF antibody titers obtained in the serum of mice immunized with TGFα-P64k protein. Only in a group of immunized mice a positive anti-EGF antibodies response is obtained.

However mice immunized with one chemical conjugated EGF-P64K didn't show any level of anti-TGFα antibodies.

### Example 18: Recognition of human tumors in vitro for a polyclonal antiserum anti-hTGFα obtained by the immunization with the TGFα-P64k protein / incomplete Adjuvant of Freund (Montanide ISA 51).

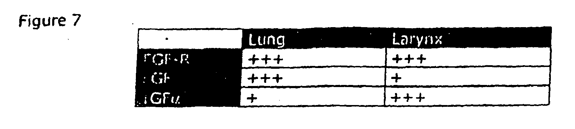

Polyclonal antiserums anti-hTGFα obtained immunizing mice with TGFα-P64k protein in Montanide ISA 51, was used to determine the TGFα expression in biopsies of tumors included in paraffin. These biopsies were obtained from patients vaccinated with a vaccine based on EGF. In a patient with high anti-hEGF antibodies response, a regression of NSCLC tumor was observed. However, later was detected a second larynx tumor. Biopsies of these two tumors were analyzed and it was observed a differential expression of EGF and TGFα in each one of them. In the figure 7 the reactivity values obtained with the different antibodies are shown. These results confirm the fact that immunization with the vaccine preparation containing TGFα-P64k provokes specific antibodies against hTGFα able to recognize this molecule in human tumors.

### Example 19: mRNA expression of EGF, TGFα and EGF-R in breast carcinoma biopsies.

Messenger ribonucleic acid (mRNA) was isolated from breast carcinoma tumor biopsies using TRIZOL reagent (Life technologies) and converted to cDNA by the reverse transcriptase enzyme. The total cDNA underwent 30 cycles of PCR using specific primers for each one of these molecules. As internal control was used a housekeeping gene (GAPDH). The PCR products obtained were separated electrophoretically on 1.5% agarosa gels and visualized with Etidium bromide.

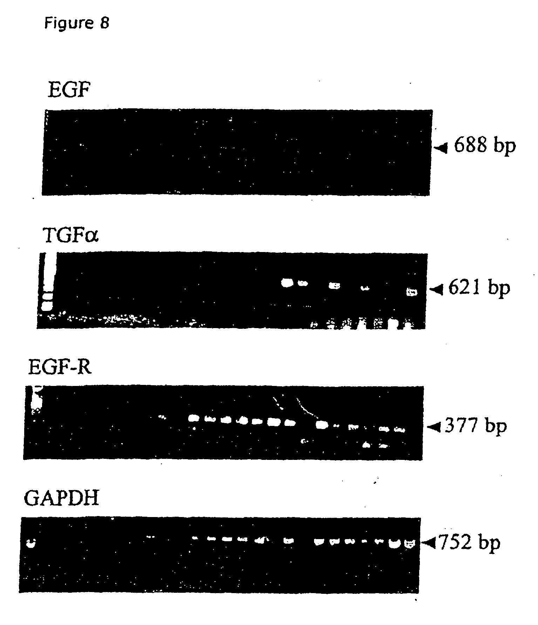

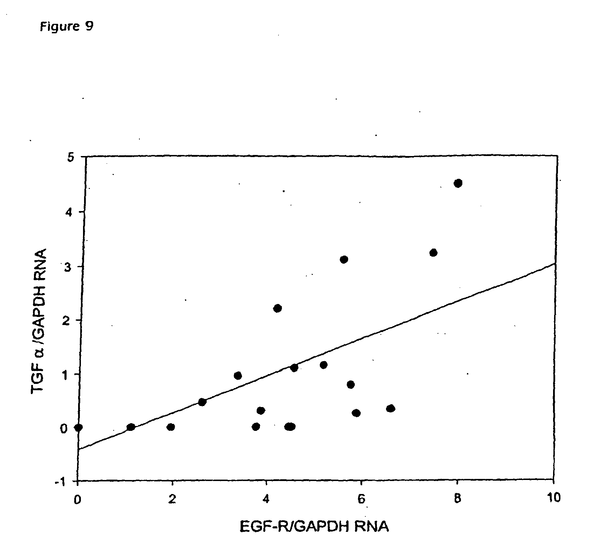

In the figure 8 are shown the results obtained using the specific primers for EGF, TGFα, EGF-R and GAPDH (internal Control) in 22 breast carcinomas. EGF mRNA was observed only in 1/22 biopsies, however it was observed a high expression of TGFα and EGF-R mRNA in most of the samples. The high correlation between the expression of these two molecules, suggests the importance of the autocrine loop TGFα / EGF-R in the growth of this type of tumors (Figure 9).

### Brief description of the drawings.

**Figure 1****:** Genetic and amino acidic sequence (letters underlined in boldface) of mature hTGFα.
**Figure 2****:** Schematic representation of expression vector pMTGFα-obtaining process.
**Figure 3****:** Recognition of TGFα-P64K fusion protein by anti-P64K Mab (A) and anti-hTGFa Mab (B) by Western Blotting technique. 10% SDS-PAGE was carried out to P64K (1), EGF-P64K (2) and TGFa-P64K (3). Then proteins were transferred to a nitrocellulose membrane and incubated with specific antibodies against P64K (A) or TGFα (B) with the objective of characterizing the fused protein between TGFα and P64K.
**Figure 4****:** Anti-hTGFα antibody response kinetics: The specific antibodies titers against hTGFα were measured by indirect ELISA'S technique. The mice were immunized with 5 µg (A), 10 µg (B) and 50 µg (C) of hTGFα -equivalent in TGFα - P64K protein mixed with Montanide ISA 51. The x-axis represent the days when the samples were collect in each mouse and the y-axis the reciprocal of the antibody titter reached. The days of immunization they are pointed out with arrows in the graph A (Day 0, 14, 28 and 42).
**Figures 5****:** IgG subclass distribution induced by the immunization with 50 µg of TGFα-equivalent in the fused protein. Comparison of IgG subclass proportion in the antibody response induced with the immunization of TGFα-P64k protein subcutaneous (1) or intramuscular (2). The values of standard deviation are shown in the figure for each one of the groups of 5 immunized animals.
**Figures 6****:** Anti-EGF specific antibodies response in mice immunized with the TGFa-P64K fused protein. In the chart are shown anti- hTGFα and anti-EGF antibody titers reached in mice immunized with TGFα -P64K.
**Figures 7****:** Determination of EGF-R, EGF and TGFα expression by immunohistochemistry in tumors biopsies of vaccinated patients included in the pilot II clinical trial of EGF-vaccine. The differential reactivity of these three molecules in the primary tumor of lung and of a second primary tumor of larynx that appeared after, are shown with positive signs in the figure.
**Figures 8****:** EGF, hTGFα and EGF-R ARNm expression in 22 breast carcinomas. The figure show the products of 30 cycles PCR obtained with specific primers for each molecule and visualized with etidium bromide after being separated on 1.5% agarosa gels. GAPDH ARNm expression, used as internal control, is also observed.
**Figures 9****:** Correlation among the hTGFα and EGF-R ARNm levels in breast carcinoma biopsies: The bands intensity obtained with etidium bromide was analyzed by means of a calculation program (ImagQuant, Amersham). The x-axis shows relation between the intensity values for the PCR products using specific primers for EGF-R and those obtained with GAPDH for each sample (relative intensity) and the y-axis the same relative intensity value for hTGFα. A positive correlation was observed between the expressions of these two molecules (R² = 0.657, p=0.00121).

## Claims

1. A vaccine composition to elicit an immune response against autologous TGF-α, which comprises as active principle a fusion protein between a carrier protein and human TGF-α or any peptide derivative of the same and additionally comprising an adjuvant, wherein said fusion protein is able to elicit said immune response against TGF-α.

2. The vaccine composition according to claim 1 wherein the carrier protein is the protein P64K from the outer membrane protein complex of *Neisseria meningitidis*.

3. The vaccine composition according to claim 2 wherein the carrier protein comprises a tail of six histidines in the N-terminal end.

4. The vaccine composition according to claim 2 wherein the vaccine composition further comprises EGF.

5. The vaccine composition according to any one of claims 1 to 4, wherein the adjuvant is Incomplete Freund Adjuvant.

6. The vaccine composition according to any one of claims 1 to 4, wherein the adjuvant is Al(OH)₃.

7. Use of a vaccine composition according to any one of claims 1 to 6 in the preparation of an agent for the treatment of malignant disease in tumours of epithelial origin which express TGF-α or EGF.

8. The use of claim 7 wherein the tumour is an epidermoide carcinoma of the lung, breast, prostate, gastric or ovary.

## Patentansprüche

1. Impfstoff-Zusammensetzung für die Erzeugung einer Immunantwort gegen autologes TGF-α, die als Wirkprinzip ein Fusionsprotein aus einem Trägerprotein und humanem TGF-α oder einem beliebigen Peptidderivat davon umfaßt, und die darüber hinaus ein Adjuvans umfaßt, wobei das Fusionsprotein in der Lage ist, die Immunantwort gegen TGF-α zu erzeugen.

2. Impfstoff-Zusammensetzung nach Anspruch 1, wobei das Trägerprotein das Protein P64K aus dem Outer Membrane-Proteinkomplex von *Neisseria meningitidis* ist.

3. Impfstoff-Zusammensetzung nach Anspruch 2, wobei das Trägerprotein einen Anhang von sechs Histidinen am N-terminalen Ende aufweist.

4. Impfstoff-Zusammensetzung nach Anspruch 2, wobei die Impfstoff-Zusammensetzung ferner EGF umfaßt.

5. Impfstoff-Zusammensetzung nach einem der Ansprüche 1-4, wobei das Adjuvans unvollständiges Freund-Adjuvans ist.

6. Impfstoff-Zusammensetzung nach einem der Ansprüche 1-4, wobei das Adjuvans Al(OH)₃ ist.

7. Verwendung einer Impfstoff-Zusammensetzung nach einem der Ansprüche 1-6 bei der Herstellung eines Mittels zur Behandlung einer malignen Erkrankung in Tumoren epithelialen Ursprungs, die TGF-α oder EGF exprimieren.

8. Verwendung nach Anspruch 7, wobei der Tumor ein epidermoidales Karzinom der Lunge, der Prostata, des Magens oder des Ovars ist.

## Revendications

1. Composition de vaccin destinée à provoquer une réponse immunitaire contre le TGF-α autologue, comprenant comme principe actif une protéine hybride entre une protéine-support et le TGF-α humain ou tout dérivé peptidique de celui-ci et comprenant en outre un adjuvant, dans laquelle ladite protéine hybride est capable de provoquer ladite réponse immunitaire contre le TGF-α.

2. Composition de vaccin selon la revendication 1, dans laquelle la protéine-support est la protéine P64K issue du complexe protéique de la membrane externe de *Neisseria meningitidis*.

3. Composition de vaccin selon la revendication 2, dans laquelle la protéine-support comprend une queue de six histidines à l'extrémité N-terminale.

4. Composition de vaccin selon la revendication 2, dans laquelle la composition de vaccin comprend en outre l'EGF.

5. Composition de vaccin selon l'une quelconque des revendications 1 à 4, dans laquelle l'adjuvant est l'adjuvant incomplet de Freund.

6. Composition de vaccin selon l'une quelconque des revendications 1 à 4, dans laquelle l'adjuvant est Al(OH)₃.

7. Utilisation d'une composition de vaccin selon l'une quelconque des revendications 1 à 6 pour la préparation d'un agent destiné au traitement d'une affection maligne dans le cas de tumeurs d'origine épithéliale exprimant le TGF-α ou l'EGF.

8. Utilisation selon la revendication 7, dans laquelle la tumeur est un carcinome épidermoïde du poumon, du sein, de la prostate, de l'estomac ou de l'ovaire.
